# EUROPEAN PATENT APPLICATION

(11) **EP 4 403 100 A1**
(43) Date of publication of application: **24.07.2024**
(21) Application number: 22870282.5
(22) Date of filing: 14.09.2022
(51) Int. Cl.: A61B 5/021, A61B 5/00, G16H 50/20

(54) **DEEP LEARNING-BASED BLOOD PRESSURE ESTIMATION SYSTEM USING PPG SIGNAL DETECTION RING**

(30) Priority: 16.09.2021 KR 20210124274
(71) Applicant: Sky Labs Inc., Seongnam-si, Gyeonggi-do 13486 (KR)
(72) Inventor: JO, Sung Mi, Seoul 08706 (KR); LEE, Min Hyung, Bucheon-si, Gyeonggi-do 14600 (KR); KIM, Chang Hyun, Seoul 05372 (KR); CHOI, Chang Woo, Uiwang-si, Gyeonggi-do 16000 (KR); LEE, Byung Hwan, Yongin-si, Gyeonggi-do 16981 (KR)
(74) Representative: Loyer & Abello
(86) International application number: PCT/KR2022/013737
(87) International publication number: WO 2023/043198

(57) **Abstract**

A deep learning-based blood pressure estimation system using a photoplethysmography (PPG) signal detection ring is provided. The system may include a server, and the server may include a signal feature extraction component configured to extract PPG features from a PPG signal by using a first deep learning model, a user feature extraction component configured to extract user features, by using a second deep learning model, from a first test PPG signal measured using the PPG signal detection ring, systolic and diastolic test blood pressures measured using a conventional blood pressure monitor simultaneously with the first test PPG signal, and user information, and a blood pressure estimation component configured to estimate systolic and diastolic blood pressures from the PPG features and the user features by using a third deep learning model, wherein the PPG signal is measured using the PPG signal detection ring, and the server receives the PPG signal from the PPG signal detection ring through a terminal.

## Description

### Technical Field

The present disclosure relates to a blood pressure estimation system. More specifically, the present disclosure relates to a deep learning-based blood pressure estimation system using a photoplethysmography (PPG) signal detection ring.

### Background Art

Blood pressure is generated by the pumping action of the heart, and blood pressure is the pressure exerted by blood on the walls of blood vessels. In general, a widely used blood pressure measurement method is an indirect blood pressure measurement method using Krotkoff sounds. First, a cuff is wrapped around the upper arm and a Korotkoff sound detector is placed. Next, the cuff pressure is increased to be above the systolic blood pressure, and then by gradually lowering the cuff pressure, Korotkoff sounds begin to be heard. The pressure at this time is systolic blood pressure. As the cuff pressure is lowered further, the Korotkoff sounds begin to decrease and the Korotkoff sounds begin to disappear. The pressure at this time is diastolic blood pressure. This conventional method of measuring blood pressure may cause the user to feel uncomfortable as it puts pressure on the arm. Additionally, it is impossible to measure blood pressure in real time with these conventional blood pressure measurement methods.

### Disclosure

### Technical Problem

An objective of the present disclosure is to provide a deep learning-based blood pressure estimation system by using a photoplethysmography (PPG) signal detection ring.

### Technical Solution

In order to solve the above-described objective, a deep-learning-based blood pressure estimation system using a photoplethysmography (PPG) signal detection ring includes a server, wherein the server includes a signal feature extraction component configured to extract PPG features from a PPG signal by using a first deep learning model, a user feature extraction component configured to extract user features, by using a second deep learning model, from a first test PPG signal measured using the PPG signal detection ring, systolic and diastolic test blood pressures measured using a conventional blood pressure monitor simultaneously with the first test PPG signal, and user information, and a blood pressure estimation component configured to estimate systolic and diastolic blood pressures from the PPG features and the user features by using a third deep learning model, wherein the PPG signal is measured using the PPG signal detection ring, and the server receives the PPG signal from the PPG signal detection ring through a terminal.

The user information may include at least one of the age, weight, height, and gender of a user.

In some embodiments, the test PPG signal, the systolic and diastolic test blood pressures, and the user information may be updated periodically.

In some embodiments, the PPG signal detection ring may include a plurality of sensors configured to simultaneously measure a plurality of PPG signals at different locations, and each of the plurality of sensors may include a light source and a photoelectric conversion device.

In some embodiments, the terminal may further include a light source control component configured to control the light source of each of the plurality of sensors such that a DC component of each second test PPG signal measured using the plurality of sensors is within a predetermined range.

In some embodiments, the terminal may further include a sensor selection component configured to select, from among the plurality of sensors, as a sensor for measuring the PPG signal, a sensor that has measured a second test PPG signal having a highest signal quality among the plurality of second test PPG signals.

In some embodiments, signal quality of the plurality of second test PPG signals may be evaluated by at least one of magnitude of an acceleration signal, a signal to noise ratio (SNR), and a ratio of an AC component magnitude to a DC component magnitude.

In some embodiments, light source control by the light source control component and sensor selection by the sensor selection component may be performed sequentially, and the light source control by the light source control component and the sensor selection by the sensor selection component may be performed periodically.

In some embodiments, the server may further include a signal quality classification component configured to classify the signal quality of the PPG signal as one of good and bad.

In some embodiments, the server may further include a blood pressure index calculation component configured to calculate a blood pressure index, wherein the blood pressure index is defined by a ratio between a time during which the quality of the PPG signal is classified as good by the signal quality classification component and the systolic blood pressure deviates from the first normal range or the diastolic blood pressure deviates from the second normal range and a time during which the quality of the PPG signal is classified as good by the signal quality classification component.

### Advantageous Effects

A deep learning-based blood pressure estimation system using a photoplethysmography (PPG) signal detection ring is provided. According to the present disclosure, there is no need to press the arm to measure blood pressure, so the user can conveniently measure blood pressure. Additionally, according to the present disclosure, blood pressure may be measured in real time.

### Description of Drawings

FIG. 1 is a three-dimensional diagram of a photoplethysmography (PPG) signal detection ring according to an embodiment of the present disclosure.
FIG. 2 is an exploded three-dimensional view of a PPG signal detection ring according to an embodiment of the present disclosure.
FIG. 3 is a block diagram of a deep learning-based blood pressure estimation system using a PPG signal detection ring according to an embodiment of the present disclosure.
FIGS. 4A and 4B are graphs showing PPG signals before preprocessing.
FIGS. 4C and 4D are graphs showing the PPG signals of FIGS. 4A and 4B after they are preprocessed, respectively.
FIGS. 5A and 5B are graphs showing PPG signals classified as good quality.
FIGS. 5C and 5D are graphs showing PPG signals classified as bad quality.
FIG. 6 is a block diagram illustrating a signal feature extraction component, a user feature extraction component, and a blood pressure estimation component.
FIG. 7 is a flowchart of a deep learning-based blood pressure estimation method using a PPG signal detection ring.
FIG. 8 is a flowchart of a method of setting a sensor of a PPG signal detection ring.

### Best Mode

### Mode for Invention

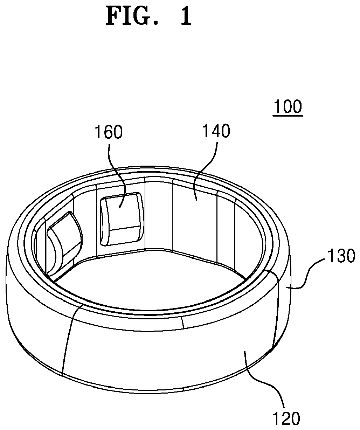

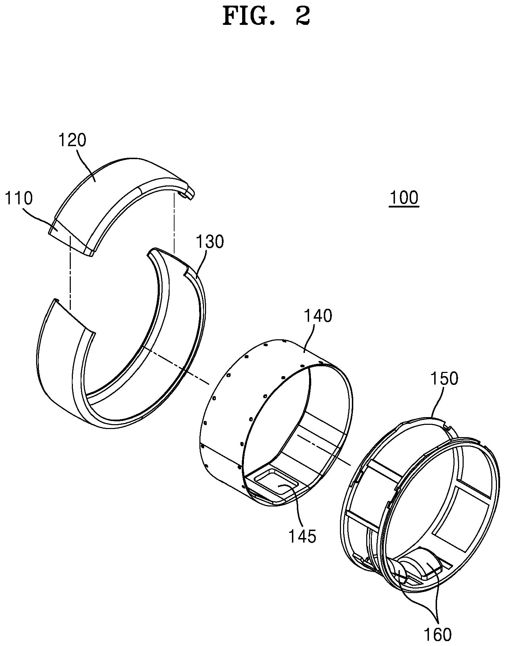

FIG. 1 is a three-dimensional diagram of a photoplethysmography (PPG) signal detection ring 100 according to an embodiment of the present disclosure. FIG. 2 is an exploded three-dimensional view of the PPG signal detection ring 100 according to an embodiment of the present disclosure.

Referring to FIGS. 1 and 2, the PPG signal detection ring 100 may include an external electrode 130, an internal electrode 140, an insulating unit 150, a top cover 110, an operation display unit 120, and a plurality of sensors 160.

The external electrode 130 may have an arc shape. The external electrode 130 may include a conductor and may function as an electrode for measuring an electrocardiogram (ECG). Additionally, the external electrode 130 may form the external appearance of the PPG signal detection ring 100, and the external electrode 130 may be in contact with the body of a user.

The internal electrode 140 may have a ring shape and may have a plurality of openings 145 for the plurality of sensors 160. The internal electrode 140 may include a conductor and may function as an electrode for measuring electrocardiogram. Additionally, the internal electrode 140 may form an inner tube of the PPG signal detection ring 100, and the internal electrode 140 may contact the finger of a user.

The insulating unit 150 may be disposed between the external electrode 130 and the internal electrode 140. The insulation unit 150 may enable electrical insulation between the external electrode 130 and the internal electrode 140.

The top cover 110 may have an arc shape and may form a ring shape together with the external electrode 130. The top cover 110 may form the external appearance of the PPG signal detection ring 100.

The operation display unit 120 may be coupled to the top cover 110. The operation display unit 120 may include a plurality of LEDs, for example, a green LED and a red LED. The operation display unit 120 may display the operation of the PPG signal detection ring 100 by using a plurality of LEDs. For example, a green LED may be turned on for two seconds to indicate the start of measurement, and a green LED may be turned on for one second to indicate the end of measurement. Additionally, the red LED may be repeatedly turned on and off at one-second intervals to notify of a malfunction.

The plurality of sensors 160 may be arranged to contact the finger of the user. The plurality of sensors 160 may be located within the plurality of openings 145 of the internal electrode 140, respectively, and may protrude from a surface of the internal electrode 140. The plurality of sensors 160 may be configured to obtain different PPG signals at different locations. Each sensor 160 may include a light source and a photoelectric conversion device.

In some embodiments, although not illustrated in FIGS. 1 and 2, the PPG signal detection ring 100 may further include an acceleration sensor disposed between the external electrode 130 and the internal electrode 140.

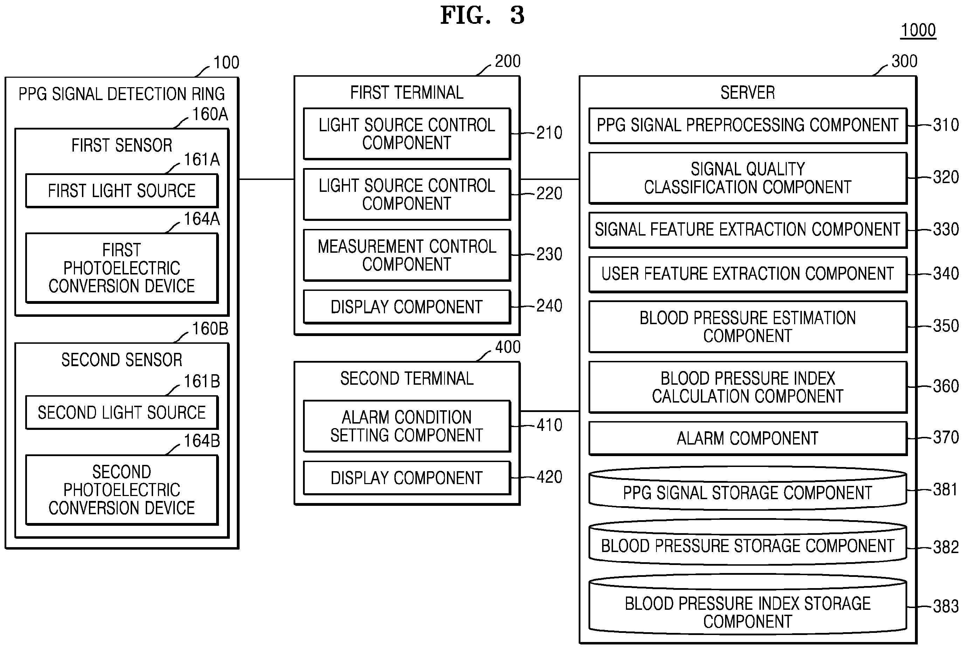

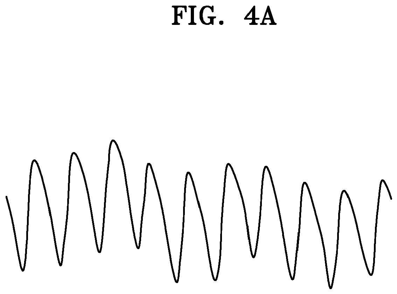

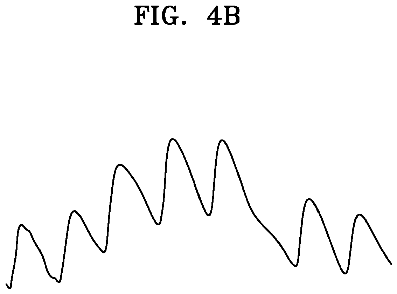

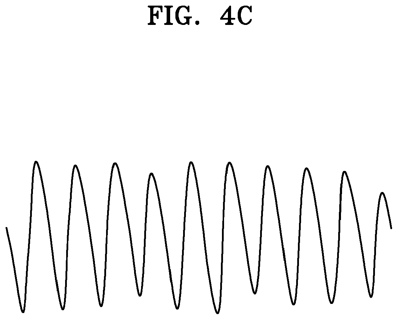

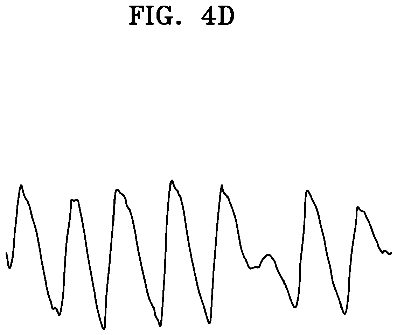

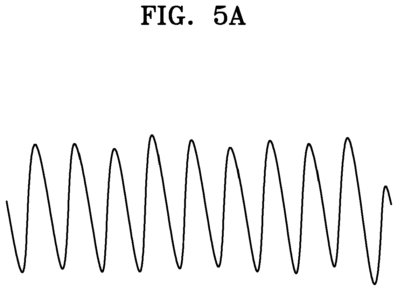

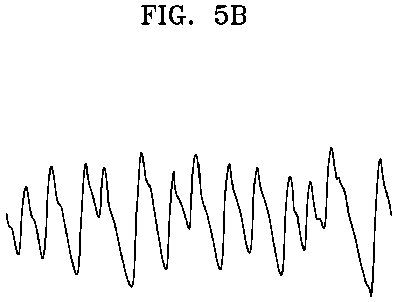

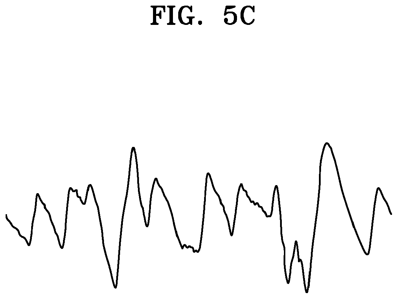

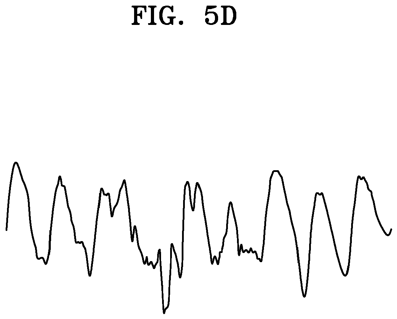

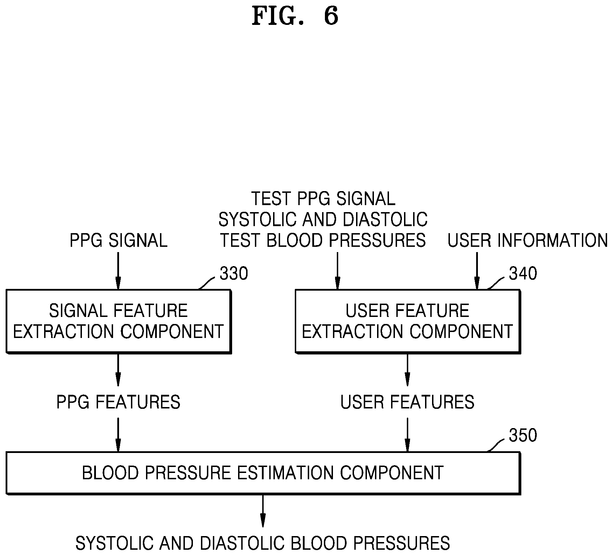

FIG. 3 is a block diagram of a deep learning-based blood pressure estimation system 1000 using a PPG signal detection ring according to an embodiment of the present disclosure. FIGS. 4A and 4B are graphs showing PPG signals before preprocessing. FIGS. 4C and 4D are graphs showing the PPG signals of FIGS. 4A and 4B after they are preprocessed, respectively. FIGS. 5A and 5B are graphs showing PPG signals classified as good quality. FIGS. 5C and 5D are graphs showing PPG signals classified as bad quality. FIG. 6 is a block diagram illustrating a signal feature extraction component 330, a user feature extraction component 340, and a blood pressure estimation component 350.

Referring to FIGS. 3, 4A to 4D, 5A to 5D, and 6, the deep learning-based blood pressure estimation system 1000 using a PPG signal detection ring may include a PPG signal detection ring 100, a first terminal 200, a server 300, and a second terminal 400.

The PPG signal detection ring 100 may include a plurality of sensors, for example, a first sensor 160A and a second sensor 160B. Although the PPG signal detection ring 100 including two sensors is illustrated in FIG. 3, the number of sensors included in the PPG signal detection ring 100 is not limited to two. In some embodiments, although not illustrated in FIG. 3, the PPG signal detection ring 100 may further include an acceleration sensor.

The first sensor 160A may include a first light source 161A and a first photoelectric conversion device 164A. The first sensor 160A may detect a first PPG signal by using the first light source 161A and the first photoelectric conversion device 164A. The first light source 161A may include, for example, a green, red, or infrared LED, and the first photoelectric conversion device 164A may include a photo diode.

The second sensor 160B may include a second light source 161B and a second photoelectric conversion device 164B. The second sensor 160B may detect a second PPG signal by using the second light source 161B and the second photoelectric conversion device 164B. The second light source 161 B may include, for example, a green, red, or infrared LED. The second light source 161B may emit light of the same wavelength as that of the first light source 161A. The second photoelectric conversion device 164B may include a photo diode.

The acceleration sensor may detect movement of the user by measuring acceleration of the PPG signal detection ring 100.

The first terminal 200 may be used by the user. The first terminal 200 may include, for example, a smartphone or a tablet PC. The first terminal 200 may be connected to the PPG signal detection ring 100 by wire or wirelessly. For example, the first terminal 200 may be connected to the PPG signal detection ring 100 by using Bluetooth or Wi-Fi. The first terminal 200 may be connected to the server 300 by wire or wirelessly. For example, the first terminal 200 may be connected to the server 300 through Wi-Fi or mobile telecommunication technology such as 3G, LTE, or 5G.

The first terminal 200 may include a light source control component 210, a sensor selection component 220, a measurement control component 230, and a display component 240. An application may be installed in the first terminal 200. The light source control component 210, the sensor selection component 220, the measurement control component 230, and the display component 240 may be implemented by an application. Alternatively, the first terminal 200 may access a website, and the light source control component 210, the sensor selection component 220, the measurement control component 230, and the display component 240 may be implemented by the website.

The light source control component 210 may control the first light source 161A and the second light source 161B such that a DC component of each of the two test PPG signals received from the first sensor 160A and the second sensor 160B satisfies a predetermined range.

After the light source control component 210 controls the first light source 161A and the second light source 161B, the sensor selection component 220 may select one of the first sensor 160A and the second sensor 160B as a sensor to measure a PPG signal. The sensor selection component 220 may select a sensor that has measured a test PPG signal with a highest signal quality among the two test PPG signals from the first sensor 160A and the second sensor 160B among the first sensor 160A and the second sensor 160B. Signal quality may be evaluated by at least one of the magnitude of an acceleration signal, a signal to noise ratio (SNR), and a ratio of an AC component magnitude to a DC component magnitude. The smaller the magnitude of an acceleration signal, the higher the signal-to-noise ratio, and the higher the ratio of the AC component magnitude to the DC component magnitude, the higher the signal quality may be. A PPG signal may then be measured by a sensor selected by the sensor selection component 220.

Control of the first light source 161A and the second light source 161B by the light source control component 210 and sensor selection by the sensor selection component 220 may be performed sequentially. That is, after the light source control component 210 controls the first light source 161A and the second light source 161B, the sensor selection component 220 may select a sensor. The control of the first light source 161A and the second light source 161B by the light source control component 210 and the sensor selection by the sensor selection component 220 may be performed periodically. The PPG signal detection ring 100 may move or rotate relative to the finger, and thus, the light source control component 210 may periodically control the light sources and the sensor selection component 220 may periodically select a sensor.

The measurement control component 230 may select a measurement mode or start or end measurement according to a user input. For example, the user may select a measurement mode between a self-check mode and a background mode by using the measurement control component 230. In the self-check mode, the user may start and end measurement by using the measurement control component 230. In the background mode, measurement starts and continues regardless of a user input. In the background mode, the user may set or change a measurement cycle. In some embodiments, the measurement control component 230 may be included in the PPG signal detection ring 100. That is, the user may select a measurement mode or start or end a measurement by using the measurement control component 230 of the PPG signal detection ring 100. In some embodiments, the measurement control component 230 may be included in the server 300. That is, a service provider may select a measurement mode or start or end measurement by using the measurement control component 230 of the server 300.

The display component 240 may display at least one of a PPG signal measured by the PPG signal detection ring 100 and stored in a PPG signal storage component 381 of the server 300, a measurement date and time of the PPG signal stored in the PPG signal storage component 381, a signal quality classification result classified by a signal quality classification component 320 of the server 300 and stored in a blood pressure storage component 382 of the server 300, blood pressure estimated by the blood pressure estimation component 350 of the server 300 and stored in the blood pressure storage component 382 of the server 300, and a blood pressure index calculated by a blood pressure index calculation component 360 of the server 300 and stored in a blood pressure index storage component 383 of the server 300.

The server 300 may include a PPG signal preprocessing component 310, the signal quality classification component 320, the signal feature extraction component 330, the user feature extraction component 340, the blood pressure estimation component 350, the PPG signal storage component 381, and the blood pressure storage component 382. In some embodiments, the server 300 may further include the blood pressure index calculation component 360 and the blood pressure index storage component 383. In some embodiments, the server 300 may further include an alarm component 370.

The PPG signal preprocessing component 310 may preprocess a PPG signal received by the server 300 through the first terminal 200 from a selected sensor of the PPG signal detection ring 100. For example, low-pass filters, high-pass filters, and normalization may be used to preprocess a PPG signal. FIGS. 4A and 4B illustrate PPG signals before preprocessing, and FIGS. 4C and 4D illustrate the PPG signals of FIGS. 4A and 4B, respectively, after they are preprocessed. In some embodiments, the PPG signal preprocessing component 310 may preprocess an acceleration signal received by the server 300 through the first terminal 200 from the acceleration sensor of the PPG signal detection ring 100.

The signal quality classification component 320 may classify the signal quality of a PPG signal as good or bad. FIGS. 5A and 5B illustrate PPG signals classified as good quality by the signal quality classification component 320. FIGS. 5C and 5D illustrate PPG signals classified as bad quality by the signal quality classification component 320. In some embodiments, the signal quality classification component 320 may refer to an acceleration signal to classify the signal quality of the PPG signal as one of good and bad.

Blood pressure estimated from a PPG signal classified to be of poor quality may be determined to be unreliable and may not be displayed by the display component 240 of the first terminal 200. Blood pressure estimated from a PPG signal classified to be of good quality may be determined to be reliable and may be displayed by the display component 240 of the first terminal 200. In an alternative embodiment, blood pressure estimated from a PPG signal may be displayed by the display component 240 of the first terminal 200 together with signal quality classification results, regardless of the signal quality classification results. Additionally, regardless of signal quality classification results, blood pressure estimated from a PPG signal may be displayed by the display component 420 of the second terminal 400, together with the signal quality classification results.

In some embodiments, the signal quality classification component 320 may use a deep learning model. The deep learning model used in the signal quality classification component 320 may include convolution neural network (CNN), Long Short-Term Memory (LSTM), Fully Connected Network (FCN), an encoder, a decoder, or a combination thereof. Methods for classifying signal quality are not limited to those described herein.

As illustrated in FIG. 6, the signal feature extraction component 330 may extract PPG features from a PPG signal by using a first deep learning model. The user feature extraction component 340 may extract user features from a test PPG signal, systolic and diastolic test blood pressures, and user information by using a second deep learning model. Systolic and diastolic test blood pressures may be measured simultaneously with the test PPG signal by using a conventional blood pressure monitor. User information may include at least one of the age, weight, height, and gender of a user. The blood pressure estimation component 350 may estimate systolic and diastolic blood pressures from PPG features and user features by using a third deep learning model. Test PPG signals, systolic and diastolic test blood pressure, and user information may be updated periodically.

Each of the first deep learning model, the second deep learning model, and the third deep learning model may include CNN, LSTM, FCN, encoder, decoder, or a combination thereof. The first deep learning model, the second deep learning model, and the third deep learning model may be trained using supervised learning. Data augmentation may be used to ensure a sufficient data set required for supervised learning.

The blood pressure index calculation component 360 may calculate a blood pressure index. A blood pressure index may be defined based on a time during which the quality of a PPG signal is classified as good by the signal quality classification component 320 and a time during which the quality of the PPG signal is classified as good by the signal quality classification component 320 and the systolic blood pressure deviates from a first normal range or the diastolic blood pressure deviates from a second normal range. For example, the blood pressure index may be defined by a ratio of a time during which the quality of a PPG signal is classified as good by the signal quality classification component 320 and the systolic blood pressure deviates from the first normal range or the diastolic blood pressure deviates from the second normal range to a time during which the quality of a PPG signal is classified as good by the signal quality classification component 320. In other words, the blood pressure index may refer to a ratio of a time when abnormal blood pressure appears to a time when reliable blood pressure estimation results may be obtained.

When at least one of blood pressure estimated by the blood pressure estimation component 350 and a blood pressure index calculated by the blood pressure index calculation component 360 satisfies an alarm condition set by an alarm condition setting component 410 of the second terminal 400, the alarm component 370 may transmit an alarm to at least one of the first terminal 200 and the second terminal 400.

The PPG signal storage component 381 may store the PPG signal received by the server 300 through the first terminal 200 from a selected sensor of the PPG signal detection ring 100. The PPG signal storage component 381 may further store a measurement date and time of the PPG signal. The PPG signal storage component 381 may further store a PPG signal preprocessed by the PPG signal preprocessing component 310. The PPG signal storage component 381 may further store an acceleration signal received by the server 300 from an acceleration sensor of the PPG signal detection ring 100 through the first terminal 200. The PPG signal storage component 381 may further store an acceleration signal preprocessed by the PPG signal preprocessing component 310. The blood pressure storage component 382 may store the blood pressure estimated by the blood pressure estimation component 350. The blood pressure storage component 382 may further store signal quality classification results classified by the signal quality classification component 320. The blood pressure index storage component 383 may store the blood pressure index calculated by the blood pressure index calculation component 360.

The second terminal 400 may be connected to the server 300 by wire or wirelessly. For example, the second terminal 200 may be connected to the server 300 through Wi-Fi or mobile telecommunication technology such as 3G, LTE, or 5G. The second terminal 400 may be used by a doctor. The second terminal 400 may include, for example, a smartphone, tablet PC, computer, or laptop.

The second terminal 400 may include the alarm condition setting component 410 and the display component 420. The second terminal 400 may be connected to a website or have an application installed. The alarm condition setting component 410 and the display component 420 may be implemented using a website or application.

Using the alarm condition setting component 410, the doctor may set alarm conditions. For example, an alarm may be set to sound when the systolic blood pressure is greater than 140 mmHg or less than 90 mmHg or the diastolic blood pressure is greater than 90 mmHg or less than 60 mmHg, for more than 10 minutes.

The display component 420 may display at least one of a PPG signal from the PPG signal storage component 381, a measurement date and time of the PPG signal from the PPG signal storage component 381, a preprocessed PPG signal stored in the PPG signal storage component 381, a signal quality classification result from the blood pressure storage component 382, blood pressure from the blood pressure storage component 382, and a blood pressure index from the blood pressure index storage component 383.

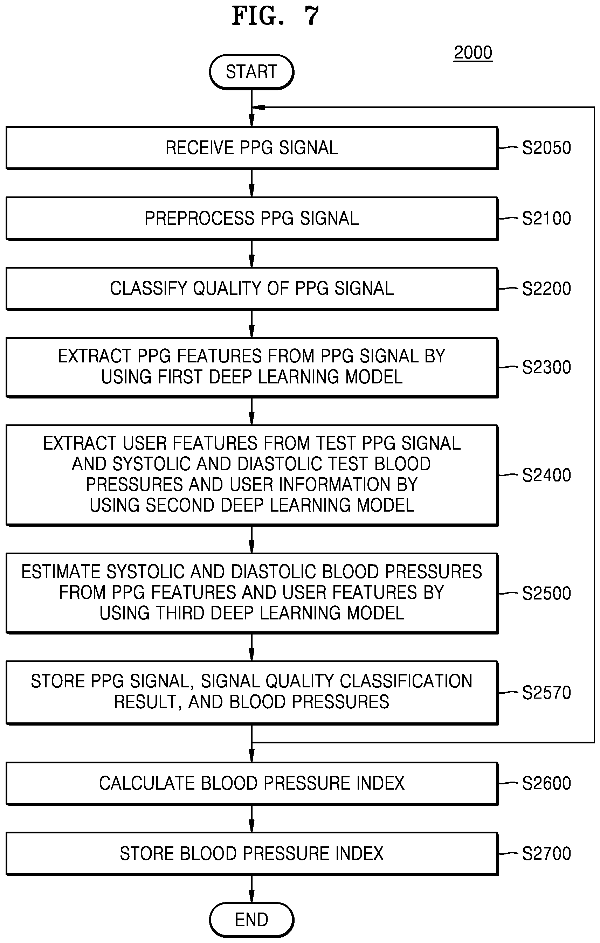

FIG. 7 is a flowchart of a deep learning-based blood pressure estimation method 2000 using a PPG signal detection ring.

Referring to FIGS. 3 and 7, the server 300 may receive a PPG signal from a selected sensor of the PPG signal detection ring 100 through the first terminal 200 (S2050). The PPG signal preprocessing component 310 may preprocess the PPG signal (S2100). For example, low-pass filters, high-pass filters, and normalization may be used to preprocess a PPG signal. FIGS. 4A and 4B illustrate PPG signals before preprocessing, and FIGS. 4C and 4D illustrate the PPG signals of FIGS. 4A and 4B, respectively, after they are preprocessed. In some embodiments, the PPG signal preprocessing component 310 may further preprocess an acceleration signal received by the server 300 through the first terminal 200 from the acceleration sensor of the PPG signal detection ring 100.

Next, the signal quality classification component 320 may classify the signal quality of the PPG signal as good or bad (S2200). The signal quality classification component 320 may refer to acceleration information to classify the signal quality of the PPG signal as good or bad. FIGS. 5A and 5B illustrate PPG signals classified as good quality by the signal quality classification component 320. FIGS. 5C and 5D illustrate PPG signals classified as bad quality by the signal quality classification component 320. Blood pressure estimated from a PPG signal classified to be of poor quality may be determined to be unreliable and may not be displayed by the display component 240 of the first terminal 200. Blood pressure estimated from a PPG signal classified to be of good quality may be determined to be reliable and may be displayed by the display component 240 of the first terminal 200.

In some embodiments, a deep learning model may be used in the signal quality classification component 320. The deep learning model used in the signal quality classification component 320 may include CNN, LSTM, FCN, encoder, decoder, or a combination thereof. Methods for classifying signal quality are not limited to those described herein.

The signal feature extraction component 330 may extract PPG features from the PPG signal by using the first deep learning model (S2300). The user feature extraction component 340 may extract user features from a test PPG signal, systolic and diastolic test blood pressures, and user information by using the second deep learning model (S2400). Systolic and diastolic test blood pressures may be measured simultaneously with the test PPG signal by using a conventional blood pressure monitor. User information may include at least one of the age, weight, height, and gender of a user. The blood pressure estimation component 350 may estimate systolic and diastolic blood pressures from PPG features and user features by using a third deep learning model (S2500). Test PPG signals, systolic and diastolic test blood pressure, and user information may be updated periodically.

Each of the first deep learning model, the second deep learning model, and the third deep learning model may include CNN, LSTM, FCN, encoder, decoder, or a combination thereof. The first deep learning model, the second deep learning model, and the third deep learning model may be trained using supervised learning. Data augmentation may be used to ensure a sufficient data set required for supervised learning.

Next, the PPG signal, signal quality classification results, and blood pressures may be stored (S2570). The PPG signal may be stored in the PPG signal storage component 381. In some embodiments, the preprocessed PPG signal may be further stored in the PPG signal storage component 381. In some embodiments, the acceleration signal may be further stored in the PPG signal storage component 381. In some embodiments, the preprocessed acceleration signal may be further stored in the PPG signal storage component 381. Signal quality classification results and blood pressures may be stored in the blood pressure storage component 382.

Next, receiving a PPG signal (S2050), preprocessing the PPG signal (S2100), classifying the quality of the PPG signal (S2200), extracting PPG features (S2300), extracting user features (S2400), estimating systolic and diastolic blood pressures (S2500), and storing the PPG signal, signal quality classification results, and blood pressures (S2570) may be repeated.

Next, the blood pressure index calculation component 360 may calculate a blood pressure index (S2600). A blood pressure index may be defined based on a time during which the quality of a PPG signal is classified as good by the signal quality classification component 320 and a time during which the quality of a PPG signal is classified as good by the signal quality classification component 320 and the systolic blood pressure deviates from a first normal range or the diastolic blood pressure deviates from a second normal range. For example, the blood pressure index may be defined by a ratio of a time during which the quality of a PPG signal is classified as good by the signal quality classification component 320 and the systolic blood pressure deviates from the first normal range or the diastolic blood pressure deviates from the second normal range to a time during which the quality of a PPG signal is classified as good by the signal quality classification component 320. In other words, the blood pressure index may refer to a ratio of a time when abnormal blood pressure appears to a time when reliable blood pressure estimation results may be obtained.

Next, the blood pressure index may be stored in the blood pressure index storage component 383 (S2700).

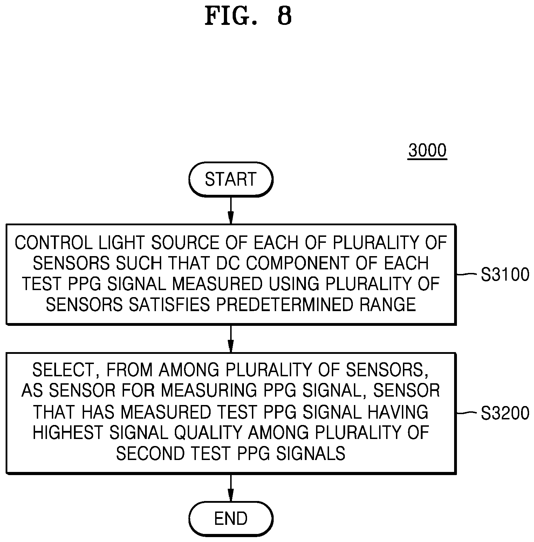

FIG. 8 is a flowchart of a method 3000 of setting a sensor of a PPG signal detection ring.

Referring to FIGS. 8 and 3, the light source control component 210 may control each light source such that a DC component of each test PPG signal measured using a plurality of sensors satisfies a predetermined range (S3100). Operation S3100 is to set each sensor, for example, the first sensor 160A and the second sensor 160B, to the best state for measuring a PPG signal.

Next, the sensor selection component 220 may select one of the plurality of sensors as a sensor for later measuring a PPG signal (S3200). The sensor selection component 220 may select a sensor that has measured a test PPG signal having a highest signal quality among two test PPG signals from the first sensor 160A and the second sensor 160B among the first sensor 160A and the second sensor 160B. Signal quality may be evaluated by at least one of the magnitude of an acceleration signal, a signal-to-noise ratio, and a ratio of an AC component magnitude to a DC component magnitude. The smaller the acceleration signal, the higher the signal-to-noise ratio, and the higher the ratio of the AC component magnitude to the DC component magnitude, the higher the signal quality may be. Thereafter, a PPG signal measured from the sensor selected by the sensor selection component 220 may be transmitted to the server 300 through the first terminal 200.

Operation of controlling a light source of each of the plurality of sensors (S3100) and operation of selecting a sensor (S3200) may be performed sequentially. Additionally, the method 3000 of setting a sensor of a PPG signal detection ring may be performed periodically. The PPG signal detection ring 100 may move or rotate relative to the finger, and thus, the light source control component 210 may periodically control the light sources and the sensor selection component 220 may periodically select a sensor.

The embodiments disclosed in the present disclosure are not intended to limit the technical idea of the present disclosure, but are for illustrative purposes, and the scope of the technical idea of the present disclosure is not limited by these embodiments. The scope of protection of the present disclosure should be interpreted in accordance with the claims below, and all technical ideas within the equivalent scope should be interpreted as being included in the scope of rights of the present disclosure.

## Claims

1. A deep-learning-based blood pressure estimation system using a photoplethysmography (PPG) signal detection ring, the system comprising a server, wherein the server comprises:
a signal feature extraction component configured to extract PPG features from a PPG signal by using a first deep learning model;
a user feature extraction component configured to extract user features, by using a second deep learning model, from a first test PPG signal measured using the PPG signal detection ring, systolic and diastolic test blood pressures measured using a conventional blood pressure monitor simultaneously with the first test PPG signal, and user information; and
a blood pressure estimation component configured to estimate systolic and diastolic blood pressures from the PPG features and the user features by using a third deep learning model,
wherein the PPG signal is measured using the PPG signal detection ring, and the server receives the PPG signal from the PPG signal detection ring through a terminal.

2. The system of claim 1, wherein the user information comprises at least one of age, weight, height, and gender of a user.

3. The system of claim 1, wherein the test PPG signal, the systolic and diastolic test blood pressures, and the user information are periodically updated.

4. The system of claim 1, wherein the PPG signal detection ring includes a plurality of sensors configured to simultaneously measure a plurality of PPG signals at different locations, and
each of the plurality of sensors includes a light source and a photoelectric conversion device.

5. The system of claim 4, wherein the terminal comprises a light source control component configured to control the light source of each of the plurality of sensors such that a DC component of each second test PPG signal measured using the plurality of sensors is within a predetermined range.

6. The system of claim 5, wherein the terminal further comprises a sensor selection component configured to select, from among the plurality of sensors, as a sensor for measuring the PPG signal, a sensor that has measured a second test PPG signal having a highest signal quality among the plurality of second test PPG signals.

7. The system of claim 6, wherein signal quality of the plurality of second test PPG signals is evaluated by at least one of magnitude of an acceleration signal, a signal to noise ratio (SNR), and a ratio of an AC component magnitude to a DC component magnitude.

8. The system of claim 6, wherein light source control by the light source control component and sensor selection by the sensor selection component are performed sequentially, and
the light source control by the light source control component and the sensor selection by the sensor selection component are performed periodically.

9. The system of claim 1, wherein the server further comprises a signal quality classification component configured to classify signal quality of the PPG signal as one of good and bad.

10. The system of claim 9, wherein the server further comprises a blood pressure index calculation component configured to calculate a blood pressure index, and
the blood pressure index is defined by a ratio between a time during which the quality of a PPG signal is classified as good by the signal quality classification component and the systolic blood pressure deviates from the first normal range or the diastolic blood pressure deviates from the second normal range and a time during which the quality of a PPG signal is classified as good by the signal quality classification component.
